(19) Europäisches Patentamt

European Patent Office

Office européen des brevets

(11) Veröffentlichungsnummer: ˙ **0 204 999**
**B1** ˙

(12) **EUROPÄISCHE PATENTSCHRIFT**

(45) Veröffentlichungstag der Patentschrift: **27.12.90**

(21) Anmeldenummer: **86106796.5**

(22) Anmeldetag: **17.05.86**

(51) Int. Cl.⁵: **A 01 K 67/00**

(54) Vorrichtung zur Aufnahme und zum Aufbewahren von Nutzorganismen.

(30) Priorität: **06.06.85 CH 2407/85**

(43) Veröffentlichungstag der Anmeldung:
**17.12.86 Patentblatt 86/51**

(45) Bekanntmachung des Hinweises auf die
Patenterteilung:
**27.12.90 Patentblatt 90/52**

(84) Benannte Vertragsstaaten:
**AT BE DE FR GB IT LU NL SE**

(56) Entgegenhaltungen:
**EP-A-0 019 168**
**BE-A- 761 115**
**DE-A-2 164 536**
**GB-A-1 196 141**
**GB-A-2 073 211**
**NL-A-7 903 776**
**US-A-3 855 727**
**US-A-4 250 833**
**US-A-4 368 690**

(73) Patentinhaber: **Züricher Beuteltuchfabrik AG**
**CH-8803 Rüschlikon (CH)**

(72) Erfinder: **Müller, Hans-Rudolf**
**Ostbühlstrasse 49**
**CH-8038 Zürich (CH)**

(74) Vertreter: **EGLI-EUROPEAN PATENT**
**ATTORNEYS**
**Horneggstrasse 4**
**CH-8008 Zürich (CH)**

Courier Press, Leamington Spa, England.

# Beschreibung

Die Erfindung betrifft eine Vorrichtung zur Aufnahme und zum Aufbewahren von Nutzorganismen nach dem Oberbegriff des Patentanspruchs 1, bekannt aus EP—A—19.168.

Bis vor kurzem erfolgte die Bekämpfung von schadenerzeugenden, bakteriellen und anderen Organismen sowie Materialien, z.B. schädlichen Abfallstoffen, fast ausschliesslich mit chemischen Materialien. Diese sind zwar sehr wirksam, jedoch kann deren Nebenwirkung je länger je weniger im Hinblick auf die damit verbundene Umweltbelastung verantwortet werden. Es werden deshalb heute umfangreiche Anstrengungen unternommen, um die Bekämpfung von bakereillen und andern schädlichen Organismen sowie von Materialien derart durchzuführen, dass eine Belastung der Umwelt möglichst vermieden wird.

Eine Anwendung dieser Art, die sich als recht aussichtsreich erwiesen hat, stellt hierbei der Einsatz von Nutzorganismen dar, d.h. von Organismen, die in der Lage sind, Schädlinge jeder Art oder Schadmaterial u.dgl. auszuschalten oder mindestens deren schädliche Wirkung zu verringern oder sogar zu neutralisieren.

Der Einsatz von Nutzorganismen setzt voraus, dass diese jeweils in einem Zeitpunkt eingesetzt werden, wo ihre Aktivität und damit ihre eigene Wirkung am grössten ist, bwz. wo die Schädlinge oder Schadstoffe am günstigsten zu beeinflussen bzw. zu vernichten sind. Dies bedeutet, dass die Nutzorganismen, bei denen es sich im allgemeinen um empfindliche, lebende Organismen handelt, unter günstigen Lebensbedingungen gehalten werden müssen.

Aus EP—A—0 019 168 ist ein mindestens teilweise mit Naturstoffen gefüllter Behälter als Unterschlupf für Nutzinsekten bekannt, der mehrere Schlupflöcher für die Insekten aufweist. Die Behälter werden an geeigneten Stellen aufgestellt und sollen Nutzinsekten zur Bleibe und Nahrungssuche in der Umgebung des Behälters veranlassen. Die Wirkung dieser Behälter ist stark abhängig von der Zahl der vorhandenen Nutzinsekten.

Aus GB—A—1 196 141 ist ein Behälter zur Aufnahme von Tieren bekannt, der von einem Netz umgeben ist, welches auch zum Abschliessen einer Eintrittsöffnung für das Tier dient. Das Netz ist als Beutel ausgebildet, welches den Behälter umgibt und mit welchem der Behälter getragen wird. Wird das Netz entfernt, kann der Behälter auch als Aufenthaltsraum für ein Haustier verwendet werden. Wegen der nur zeitweiligen Verwendung des Netzes als Abschluss des Behälters ist es für die Aufnahme von Nutzorganismen — auch wegen seiner Grösse — nicht geeignet.

Der Erfindung liegt die Aufgabe zugrunde, eine Vorrichtung der eingangs erwähnten Art so auszugestalten, dass für die einzusetzenden Nutzorganismen sowohl beste Voraussetzungen für ihre Entwicklung als auch für ihren zeitlichen Einsatz erreicht werden, durch die Vorrichtung selbst aber keine Belastung der Umwelt entsteht.

Diese Aufgabe wird gemäss der Erfindung durch das Kennzeichen des Patentanspruchs 1 gelöst. Dadurch wird erreicht, dass ein ausreichender Schutz für die Entwicklung der Nutzorganismen und eine Selektion bezüglich der Grösse dieser Nutzorganismen erreicht wird.

Die Erfindung ist in der Zeichnung beispielsweise dargestellt und nachfolgend beschrieben. Es zeigen:

Fig. 1 ein zylinderförmiges Gehäuse mit einem Aufhänger und einer getrennt dargestellten Abdeckung mit kalibrierten Oeffnungen in räumlicher Darstellung.

Fig. 2 eine schematisch dargestellte Seitenansicht eines pyramidenstumpfförmigen Gehäuses mit Abdeckung,

Fig. 3 einen schematisch dargestellten Querschnitt einer konusförmigen Abdeckung mit einer kalibrierten Oeffnung,

Fig. 4 eine schematisch dargestellte Seitenansicht eines Einsatzes für eine Gehäuse mit etagenförmiger Anordnung verschiedener, definierter Oeffnungen,

Fig. 5 einen schematisch dargestellten Querschnitt einer deckelförmigen Abdeckung mit einem Siebgewebe definierter Maschenweite für ein Gehäuse,

Fig. 6 einen schematisch dargestellten Querschnitt einer weiteren Abdeckung mit definierten Oeffnungen für ein Gehäuse,

Fig. 7 eine schematisch dargestellte Seitenansicht eines kugelförmigen Gehäuses mit einem Einsteckpfahl und einem Sonnenschutz,

Fig. 8 einen schematisch dargestellten Querschnitt einer weiteren Austuhrungsform eines Sonnenschutzes für ein Gehäuse und

Fig. 9 einen schematisch dargestellten Querschnitt eines zylinderförmigen Gehäuses mit einem hohlen, zylinderförimigen Einsatz.

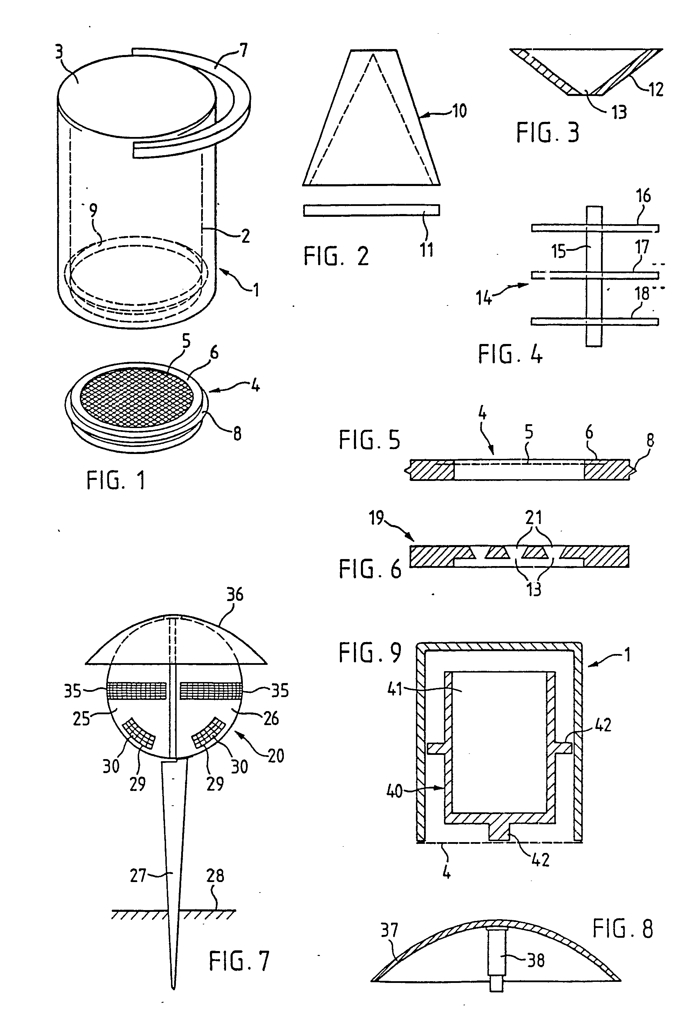

Die Erfindung geht von der Ueberlegung aus, dass den Nutzorganismen, die für die Schädlings- und Schadstoff-Bekämpfung eingesetzt werden sollen, einerseits ein ausreichender Schutz geboten wird und andererseits die Nutzorganismen in einem für den Einsatz günstigen Zeitraum eingesetzt werden können. In den Figuren 1—9 sind Beispiele von Vorrichtungen dargestellt, mit denen der richtige Einsatz der Nutzorganismen erreicht wird.

In Fig. 1 ist mit 1 ein Gehäuse bezeichnet, das als zylinderförmiger Hohlkörper 2 ausgebildet ist und an einem Ende durch einen Boden 3 abgeschlossen ist, während an dem andern Ende eine Abdeckung 4 aufgesetzt wird. Die Abdeckung 4 ist hier als Sieb ausgebildet, dessen Siebgewebe 5 eine definierte Maschenweite aufweist. Das Siebgewebe 5 ist in einem kreisringförmigen Rahmen 6 befestigt.

Im Hohlraum des Gehäuses 1 werden Nutzorganismen oder die Brut solcher Nutzorganismen eingebracht und hierauf das Gehäuse 1 mit der Abdeckung 4 geschlossen. Durch die definierte Maschenweite wird erreicht, dass nur Nutzorganismen das Gehäuse 1 verlassen können, die einen kleineren Körperquerschnitt aufweisen als die Querschnittfläche der Maschenweite. Das Gehäuse 1 stellt auch einen ausreichenden Schutz für die Nutzorganismen dar. Damit das Gehäuse 1 am Einsatzort mit nach unten gerichteter Abdeckung 4 angeordnet werden kann, ist am Gehäuse 1 ein Aufhänger 7, in Fig. 1 in Form eines Bügels, angeordnet, der ausser der Anordnung in Fig. 1 auch oben angebracht sein kann.

Das Gehäuse 1 ist zweckmässig aus einem Kunststoff hergestellt. Damit die Abdeckung 4 mit dem Gehäuse 1 zuverlässig verbunden ist, wird sie mit Hilfe eines Schnappverschlusses in das Gehäuse 1 eingesetzt. Dieser besteht aus einem ringförmigen Vorsprung 8 auf der Umfangsfläche des Rahmens 6 der Abdeckung 4 und einem an der Innenwand des Hohlkörpers 2 angeordneten nutenförmigen Rücksprunges 9. Der Rahmen 6 und auch die Fäden des Gewebes 5 bestehen vorzugsweise aus Kunststoff.

Est ist nun wesentlich, dass für die Kunststoffteile ein Kunststoff gewählt wird, der während der Zeit des Einsatzes der Schutzorganismen intakt ist, aber nachher zerfällt, wobei die Zerfallprodukte keine umweltbelastenden Komponenten, z.B. Gifte, enthalten dürfen. Hierzu eignet sich beispielsweise ein Polyamide, das einen proteinähnlichen Aufbau aufweist.

Damit der verwendete Kunststoff bzw. die verwendeten Kunststoffe nach Beendigung des Einsatzes von Nutzorganismen nicht zu lange noch intakt bleiben, können das Gehäuse 1 und die Abdeckung 4 vorbehandelt werden. Mit einer solchen Vorbehandlung, die beispielsweise in einer Bestrahlung, z.B. durch Untraviolett, durch β-Strahlen o.dgl., bestehen kann, kann die Lebensdauer, d.h. die Dauer de Intaktheit des Kunststoffes dosiert werden. Anstelle der genannten Bestrahlungen ist jedoch auch eine chemische Behandlung, z.B. mit oxydierenden Chemikalien, möglich, um die Lebensdauer der Kunststoffe des Gehäuses 1 und der Abdeckung 4 zu dosieren. Anstelle von Polyamiden als Kunststoff ist auch die Verwendung von Zellulosekunststoffen, Polyäthylen, Polypropylen, Polyester o.dgl. möglich, die ebenfalls ohne Umweltbelastung zerfallen und in ihrer Lebensdauer einstellbar sind.

Das Gehäuse 1 kann auch bezüglich der Farbe so gewählt werden, dass der Schutz der Nutzorganismen gewährleistet ist. Die Farbe kann beispielsweise reflektieren oder auch opak sein.

In den Figuren 2—9 sind Varianten dargestellt, die einerseits die Form des Gehäuses und andererseits die Ausbildung der definierten Oeffnungen betreffen.

In Fig. 2 ist das Gehäuse kegel- oder pyramidenstumpfförmig ausgebildet. Die dazugehörige Abdeckung 11 kann ähnlich aufgebaut sein wie die Abdeckung 4 in Fig. 1. Sie kann jedoch auch als Kunststoffteil gespritzt werden, wobei die definierten Oeffnungen in der Spritzform vorgesehen sind.

In Fig. 3 ist schematisch eine kegelförmige Abdeckung 12 dargestellt, die nur eine einzige definierte Oeffnung 13f aufweist.

In Fig. 4 ist eine als Einsatz in das Gehäuse einzusetzende Mehrfachabdeckung 14 schematisch dargestellt, die aus einem stabförmigen Halter 15 und mehreren, in Fig. 4 drei, scheibenförmigen Abdeckungen 16, 17, 18 zusammengesetzt ist. Die Abdeckungen 16, 17, 18 weisen die definierten Oeffnungen (nicht dargestellt) auf, wobei in jeder dieser Abdeckungen definierte Oeffnungen unterschiedlicher Grösse vorgesehen werden können. Ist die Abdeckung 16 die innenliegende Abdeckung, kann diese die kleinsten Oeffnungen, z.B. etwa 100 µm, aufweisen, während die nach aussen daran anschliessenden Abdeckungen 17, 18 zunehmend grössere Oeffnungen aufweisen können, z.B. bis etwa 500 µm.

In Fig. 5 ist die Abdeckung 4, wie sie bei dem Gehäuse 1 nach Fig. 1 verwendet wird, dargestellt. Das Siebgewebe 5 mit definierter Maschenweite ist in dem aus Kunststoff gespritzten Rahmen 6 eingebettet. Am Aussenumfang des Rahmens 6 ist der Vorsprung 8 der Schnappvorrichtung sichtbar.

In Fig. 6 ist eine aus Kunststoff gespritzte Abdeckung 19 mit darin angeordneten definierten Oeffnungen 13 dargestellt. Die Oeffnungen 13 liegen am Ende konisch zulaufender Vertiefungen 21.

In Fig. 7 ist ein kugelförmiges Gehäuse 20 dargestellt, das aus zwei zusammengefügten Halbschalen 25, 26 zusammengefügt ist. Die Verbindung der beiden halbkugeligen Schalen 25, 26 kann ebenfalls mit einer Schnappverbindung, wie sie in Verbindung mit Fig. 1 beschrieben ist, erreicht werden. Die Halbschale 26 ist mit einem Pfahl 27 versehen, mit dem das Gehäuse im Boden 28 eingesteckt werden kann. Anstelle des Pfahls kann auch ein Stativ vorgesehen werden, mit dem das Gehäuse aufgestellt werden kann. Die Halbschalen 25, 26 weisen Wandteile 29 mit Abdeckungen 30 auf. Die Abdeckungen 30 sind z.B. gemäss den Ausführungsformen nach Fig. 5 und 6 ausgebildet. Während die Abdeckungen 30 auf der unteren Hälfte des Gehäuses 20 liegen, sind im Bereich des grössten Durchmessers Fenster 35 vorgesehen, die der Belüftung des Hohlraumes des Gehäuses 20 dienen. Die Fenster 35 sind hierbei mit einem Siebgewebe abgedeckt, dessen Maschenweite kleiner ist als diejenige der Abdeckungen 30.

Ein weiteres, dem Schutz der Nutzorganismen dienendes Mittel stellt ein in Fig. 7 dargestellter Sonnenschutz 36 dar. Der Sonnenschutz 36 breitet sich haubenförmig über das Gehäuse 20 aus und überragt dasselbe seitlich. Ein ähnlicher Sonnenschutz 37 ist in Fig. 8 dargestellt, der eine Stütze 38 aufweist, mit Hilfe er mit Abstand auf das Gehäuse 20 aufgesetzt werden kann.

In Fig. 9 ist ein Gehäuse 1 gemäss Fig. 1 dargestellt, bei dem aber die Abdeckung 4 nur schematisch dargestellt ist und nach einer der beschriebenen Ausführungen ausgebildet sein kann. In dem Gehäuse 1 ist ein Einsatz 40 eingelegt, der als Gehäuse mit einem Hohlraum 41 ausgebildet ist. Im Hohlraum 41 können die Nutzorganismen untergebracht sein. Mit Abstützungen 42 kann die Lage des Einsatzes 40 fixiert werden.

Anstelle des Einsatzes 40 können auch andre Formen von Einsätzen verwendet werden, z.B. Blenden oder Barrieren, mit denen der Innenraum der Gehäuse unterteilt werden kann, ohne jedoch definierte Oeffnungen zu bilden. Diese müssen in Abdeckungen vorgesehen werden, wie sie beispielsweise in Fig. 4 dargestellt sind.

Die beschriebene Vorrichtung weist den Vorteil auf, dass sie viele Möglichkeiten eröffnet, wie die Unterbringung von Nutzorganismen optimal durchgeführt werden kann. Hierzu kann die Form der Gehäuse, ihre Farbe und ihr Material in weitem Umfang angepasst werden. Auch können zusätzliche Mittel eingesetzt werden, mit denen ein zusätzlicher Schutz erreicht wird, z.B. durch Aufhänger, Sonnenschutz und Einsätzen im Innern der Gehäuse.

**Patantansprüche**

1. Vorrichtung zur Aufnahme und zum Aufbewahren von Nutzorganismen für die Beeinflussung der Umwelt, insbesondere für die Bekämpfung und Vernichtung von schadenerzeugenden bakteriellen und andern Organismen sowie Materialien, wobei die Nutzorganismen in einem als Hohlkörper (2) ausgebildeten Gehäuse (1, 10, 20) untergebracht sind, dadurch gekennzeichnet, dass das Gehäuse (1, 10, 20) mindestens einen mit einer Abdeckung (4, 11, 12, 14, 16—18, 19, 30) versehenen Wandteil (29) aufweist, in welchem mindestens eine Oeffnung (13) angeordnet ist, welche in ihren Dimensionen so definiert ist, daß nur Nutzorganismen mit kleinerer Körper-Querschnittfläche das Gehäuse verlassen können, wobei das Gehäuse aus einem Kunststoff mit nichttoxischen Eigenschaften aus einem Polyamid, Zellulosekunststoff, Polyäthylen, Polypropylen oder Polyester hergestellt ist, dessen Intaktheitsdauer bis zu seiner Verwitterung oder zu seinem Verfall durch eine chemische oder physikalische Behandlung zeitlich eingestellt ist und dessen Verwitterungs- oder Zerfallsprodukte ebenfalls nichttoxische Eigenschaften aufweisen.

2. Vorrichtung nach Anspruch 1, dadurch gekennzeichnet, dass die Abdeckung (4, 19, 30) mehrere Oeffnungen aufweist, welche die Maschen eines Siebgewebes mit definierter Maschenweite bilden.

3. Vorrichtung nach Anspruch 1 oder 2, dadurch gekennzeichnet, dass die Abdeckung (4, 19) ein vom Gehäuse (1) getrennter, beispielsweise einschnappbarer Gehäuseteil ist, in welchem die Oeffnung bzw. die Oeffnungen (13) angeordnet ist bzw. sind.

4. Vorrichtung nach Anspruch 1, dadurch gekennzeichnet, dass das Gehäuse (1, 10, 20) eine, mindestens eine Symmetrieebene aufweisende Form, z.B. Quaderform, Zylinderform, Kegelform, Kugelform, Pyramiden- und Pyramidenstumpfform, aufweist.

5. Vorrichtung nach Anspruch 1, dadurch gekennzeichnet, dass das Gehäuse (20) mindestens ein Fenster (35) zur Belüftung des Gehäuses aufweist, deren Abdeckung mit kleineren Durchgängen versehen ist als die in ihren Dimensionen definierten Oeffnungen (13) in der Abdeckung.

6. Vorrichtung nach Anspruch 1, dadurch gekennzeichnet, dass das Gehäuse (20) mit zusätzlichen, dem Schutz der Nutzorganismen dienenden Mitteln, z.B. einem Aufhänger (7), einem Aufsteckpfahl (27), einem Sonnenschutz (36, 37) o.dgl., versehen ist.

7. Vorrichtung nach Anspruch 1, dadurch gekennzeichnet, dass in dem Hohlraum (2) des Gehäuses (1) mindestens ein Einsatz, z.B. Blenden, Regale mit Oeffnungen, Hohlkörper (40) o.dgl., eingebracht sind.

**Revendications**

1. Dispositif pour contenir et garder des organismes utiles pour influencer sur l'environnement, notamment pour la lutte et la destruction d'organismes bactériens et autres ainsi que des matières nocives, les organismes utiles étant logés dans un boîtier (1, 10, 20) réalisé en tant que corps creux (2), caractérisé en ce que le boîtier (1, 10, 20) présente une partie de paroi (29) munie d'au moins un couvercle (4, 11, 12, 14, 15—18, 19, 30), dans lequel est prévu au moins une ouverture (13) définie, quant à ses dimensions, de façon à ce que seuls des organismes utiles dont le corps présente une section transversale plus petite puissent quitter le boîtier, le boîtier étant réalisé en une matière plastique dont les propriétés ne sont pas toxiques en une polyamide, une matière plastique de cellulose, un polyéthylène, un polypropylène ou un polyester, dont la durée d'intégrité est réglée dans le temps jusqu'à sa dégradation par l'atmosphère ou sa décomposition par un traitement chimique ou physique et dont les produits de décomposition par l'atmosphère ou de désintégration présentent également des propriétés non toxiques.

2. Dispositif suivant la revendication 1, caractérisé en ce que le courvercle (4, 19, 30) présente plusieurs ouvertures formant les mailles d'un filtre tissé à largeur de mailles définie.

3. Dispositif suivant la revendication 1 ou 2, caractérisé en ce que le couvercle (4, 19) constitue un élément de boîtier, dans lequel l'ouverture ou les ouvertures (13), sont prévues, ledit couvercle étant séparé du boîtier (1), auquel il peut par exemple être encliqueté.

4. Dispositif suivant la revendication 1, caractérisé en ce que le boîtier (1, 10, 20) revêt une forme présentant au moins un plan de symétrie, par exemple, une forme de parallélépidède, une forme cylindrique, une forme conique, une forme sphérique, une forme pyramidale et une forme de tronc de pyramide.

5. Dispositif suivant la revendication 1, caractérisé en ce que le boîtier (20) présente au moins une ouverture (35) pour l'aération du boîtier, dont le couvercle présente des passages plus petits que les ouvertures (13) du couvercle dont les dimensions sont définies.

6. Dispositif suivant la revendication 1, caractérisé en ce que le boîtier (20) comporte des moyens supplémentaires servant à la protection des organismes utiles, par exemple, un élément de suspension (7), un piquet de mise en place (27), un écran solaire (36, 37) ou analogue.

7. Dispositif suivant la revendication 1, caractérisé en ce qu'au moins un insert, par exemple, diaphragmes, étagères avec ouvertures, corps creux (40) ou analogues sont placés dans l'espace creux (2) du boîtier (1).

**Claims**

1. Device for receiving and storing useful organisms for influencing the environment, particularly for combating and eliminating damage-generating bacterial and other organisms as well as materials, wherein the useful organisms are introduced into housing (1, 10, 20) constructed as a hollow body (2), characterised in that the housing (1, 10, 20) has at least one wall part (29) provided with a cover (4, 11, 12, 14, 16—18, 19, 30) in which is arranged at least one opening (13) which is so defined in its dimensions that only smaller body cross sectional area useful organisms can leave the housing, wherein the housing is manufactured of a plastics with non-toxic properties of a polyamide, cellulose plastic, polyethylene, polypropylene or polyester, the intactness period of which up to its weathering or to its disintegration is adjusted in terms of time by a chemical or physical treatment and the weathering or decomposition products of which likewise have non-toxic properties.

2. Device according to Claim 1, characterised in that the cover (4, 19, 30) has several openings which form the meshes of a sieve cloth with defined mesh width.

3. Device according to Claim 1 or 2, characterised in that the cover (4, 19) is a housing part separate from the housing (1), for example, snappable in, in which the opening or the openings (13) is or are arranged.

4. Device according to Claim 1, characterised in that the housing (1, 10, 20) has a shape having at least one plane of symmetry, e.g. square shape, pyramidal or truncated pyramidal shape.

5. Device according to Claim 1, characterised in that the housing (20) has at least one window (35) for aerating the housing, the cover of which is with smaller passages than the openings of defined dimension (13) in the cover.

6. Device according to Claim 1, characterised in that the housing (20) is provided with additional means serving for protection of the useful organisms, e.g. a hanger (7), a sticking-in spike (27), a sun protector (36, 37) or the like.

7. Device according to Claim 1, characterised in that in the hollow space (2) of the housing (1), there is introduced at least one insert, e.g. shutters, shelves with openings, hollow bodies (40) or the like.

FIG. 1

FIG. 2

FIG. 3

FIG. 4

FIG. 5

FIG. 6

FIG. 7

FIG. 8

FIG. 9